# EUROPEAN PATENT APPLICATION

(11) **EP 1 380 307 A1**
(43) Date of publication of application: **14.01.2004**
(21) Application number: 02717144.6
(22) Date of filing: 15.04.2002
(51) Int. Cl.: A61K 45/00, A61K 31/27, A61K 31/421, A61K 31/422, A61P 1/16, A61P 43/00, A61P 11/00, C07D 263/32, C07D 413/04

(54) **TISSUE FIBROSIS INHIBITORS**

(30) Priority: 18.04.2001 JP 2001119601
(71) Applicant: FUJISAWA PHARMACEUTICAL CO., LTD., Osaka-shi Osaka 541-8514 (JP)
(72) Inventor: MAEDA, Noriaki, Fujisawa Pharmaceutical Co., Ltd., Osaka-shi, Osaka 541-8514 (JP); NAGAKURA, Y., Fujisawa Pharmaceutical Co., Ltd., Osaka-shi, Osaka 541-8514 (JP); OTA, Mariko, Fujisawa Pharmaceutical Co., Ltd., Osaka-shi, Osaka 541-8514 (JP); HIRAYAMA, Y., Fujisawa Pharmaceutical Co., Ltd., Osaka-shi, Osaka 541-8514 (JP); SASAKAWA, T., Fujisawa Pharmaceutical Co., Ltd., Osaka-shi, Osaka 541-8514 (JP); OE, Tomoya, Fujisawa Pharmaceutical Co., Ltd., Osaka-shi, Osaka 541-8514 (JP)
(74) Representative: Gille Hrabal Struck Neidlein Prop Roos
(86) International application number: PCT/JP2002/003714
(87) International publication number: WO 2002/085412

(57) **Abstract**

The invention provides a pharmaceutical composition containing, as the active ingredient thereof, a prostaglandin-I₂ agonist, therefore providing a remedy or preventive for various disorders resulting from tissue fibrogenesis.

## Description

### TECHNICAL FIELD

The present invention relates to a tissue fibrogenesis inhibitor containing, as the active ingredient thereof, a prostaglandin-I₂ agonist.

### BACKGROUND ART

It is known that the progress in tissue fibrogenesis in liver, lung, kidney, pancreas, skin and others worsens various disorders of those organs.

For example, regarding hepatic disorders caused by hepatitis virus infection, the hepatitis virus infection first brings about acute hepatitis, and the patient who could not completely exterminate the viruses are continuously infected with the viruses and therefore have chronic viral hepatitis. In those with chronic viral hepatitis, the liver cells are continuously damaged and regenerated. Owing to the liver-regenerating mechanism, most cases of chronic hepatitis may keep a sufficient liver function.

However, the continuous chronic hepatitis brings about repetitive connective tissue propagation, which is naturally considered as a curing process after inflammation-caused tissue necrosis. As a result, hepatic fibrogenesis will progress in liver, and it will then lead to cirrhosis. The progress in cirrhosis gradually worsens the liver function, and finally it leads to hepatic insufficiency to death. In addition to such chronic viral hepatitis, it is known that alcoholic- or drug-induced, or autoimmunity-derived chronic hepatitis will follow the same process. In particular, cirrhosis caused by the progress in viral hepatitis brings about an extremely high frequency of hepatoma generation.

Accordingly, if the progress toward cirrhosis could be prevented, then long-term survival of patient who suffer from chronic hepatitis and keep a sufficient liver function may be expected. Since hepatoma often follows cirrhosis as a complication thereof, the prevention of cirrhosis will lead to the prevention of hepatoma. Namely, the prevention of cirrhosis is the best therapy for the cases of chronic hepatitis. Therefore, remedies for promoting the regeneration of liver cells necrosed by hepatitis, or liver fibrogenesis inhibitors are desired.

It is believed that tissue fibrogenesis in lower airways typically in lung will occur and progress as a result of abnormal tissue restoration after tissue injury by various factors. The fibrogenesis in lung and airway tissues, which is caused by interstitial pneumonia with clear exciting causes (pneumoconiosis, hypersensitivity pneumonitis, drug-induced and radiation-induced pneumonitis, etc.), interstitial pneumonia without clear exciting causes (idiopathic interstitial pneumonia, interstitial pneumonia with collagenosis, sarcoidosis, etc.), acute respiratory distress syndromes (ARDS), cystic fibrosis of the lung, chronic obstructive pulmonary diseases or the like, damages the respiratory function and may often menace lives. Accordingly, there is a possibility that the substances capable of inhibiting lung tissue fibrogenesis could be preventives or remedies for these disorders.

Further, even in the tissue of kidney, pancreas or skin, the tissue fibrogenesis leads to serious disorders such as nephrosclerosis,pancreaticfunctioninsufficiency,pachyderma, etc. The substances capable of inhibiting tissue fibrogenesis are expected to be effective also for these disorders.

It is known that the prostaglandin-I₂ agonist used in the present invention has pharmaceutical effects for inhibition of platelet aggregation, vasolidation, antihypertension, etc., and is effective for cerebrovascular accident, arterial obstruction, arteriosclerosis, obstructive arteriosclerosis (including various symptoms such as intermittent claudication, resting pain, etc.), thromboembolism, ischemic cardiopathy, restenosis after percutaneous arterioplasty, hypertension (including pulmonary hypertension) , gastric ulcer, skin ulcer, etc. (e.g., WO95/17393, WO95/24393, WO00/78350).

In addition to the above applications, it is said that prostaglandin-I₂ agonist is also effective for hepatic disorders such as hepatitis, hepatic insufficiency (WO93/07876, WO95/17393). For example, it is known that beraprost, a type of prostaglandin-I₂ agonist, is effective for inhibiting fibrin storage, and it has been clarified that it can be used for remedies for hepatitis as it inhibits hepatic coagulation necrosis in acute hepatitis (*Dig. Dis. Sci.* 40, pp. 41-99 (1995)) . However, no one knows that prostaglandin-I₂ agonist may be effective for antifibrogenesis, especially for inhibition of hepatic fibrogenesis.

### DISCLOSURE OF THE INVENTION

In consideration of the current circumstance as mentioned above, an object of the invention is to provide a tissue fibrogenesis inhibitor which has an effect of inhibiting tissue fibrogenesis and which is therefore effective for treatment or prevention of various organ disorders caused by the progress in tissue fibrogenesis in liver, lung, kidney, pancreas, skin, etc.

We, the present inventors have investigated substances that may inhibit fibrogenesis in liver or lung, an organ in which the tissue fibrogenesis leads to serious disorders, and, as a result, have found that prostaglandin-I₂ agonist is effective for inhibiting tissue fibrogenesis and have completed the present invention.

Specifically, the pharmaceutical composition containing prostaglandin-I₂ agonist, which the invention provides herein, is characterized in that it inhibits hepatic fibrogenesis resulting from chronic hepatopathy and prevents cirrhosis. For lung, the pharmaceutical composition is characterized in that it inhibits fibrogenesis in lung tissue and therefore it is effective for treatment and prevention of interstitial pneumonia and fibroid lung (for example, resulting from pulmonary emphysema).

Moreover, prostaglandin-I₂ agonist is effective for inhibiting tissue fibrogenesis, and the invention also provides a tissue fibrogenesis inhibitor effective for treatment or prevention of various organdisorders (for example, pancreatitis with fibrogenesis, nephritis with fibrogenesis) caused by the progress in tissue fibrogenesis in kidney, pancreas, skin, etc.

Specifically, the invention provides the following:
[1] A pharmaceutical composition for treatment or prevention of disorders with tissue fibrogenesis in humans and animals, which contains, as the active ingredient thereof, a prostaglandin-I₂ agonist.
[2] Amethod for inhibiting tissue fibrogenesis, which comprises administering a therapeutically effective amount of a prostaglandin-I₂ agonist to a patiant with tissue fibrogenesis.
[3] A method of using a prostaglandin-I₂ agonist in producing a pharmaceutical composition for treatment or prevention of disorders with tissue fibrogenesis.
[4] A pharmaceutical composition for treatment or prevention of hepatic disorders with fibrogenesis in humans and animals, which contains, as the active ingredient thereof, a prostaglandin-I₂ agonist.
[5] A pharmaceutical composition for prevention of cirrhosis or hepatic insufficiency in humans and animals, which contains, as the active ingredient thereof, a prostaglandin-I₂ agonist.
[6] A pharmaceutical composition for treatment or prevention of lung disorders with fibrogenesis in humans and animals, which contains, as the active ingredient thereof, a prostaglandin-I₂ agonist.
[7] A pharmaceutical composition for treatment or prevention of fibroid lung in humans and animals, which contains, as the active ingredient thereof, a prostaglandin-I₂ agonist.

### BEST MODES OF CARRYING OUT THE INVENTION

The active ingredient, prostaglandin-I₂ agonist for use in the invention includes, for example, a series of drugs capable of specifically binding with the IP receptor that exists in fibroblasts, inflammatory cells, Ito cells and the like, to thereby induce a PGI₂ [(5Z,9α,11α,13E,15S)-6,9-epoxy-11,15-dihydroxyprosta3,15-d ien-1-onic acid]-like action. It is preferably a nonprostanoid-prostaglandin-I₂ agonist with none of prostaglandin skeleton, bicyclo[3,3,0]octane skeleton nor 2-oxabicyclo[3,3,0]octane skeleton.

More preferred examples of the prostaglandin-I₂ agonist for use in the invention are the compounds of the following formula (I), (II) or (III) or their pharmaceutically-acceptable salts. Compounds of formula (I): 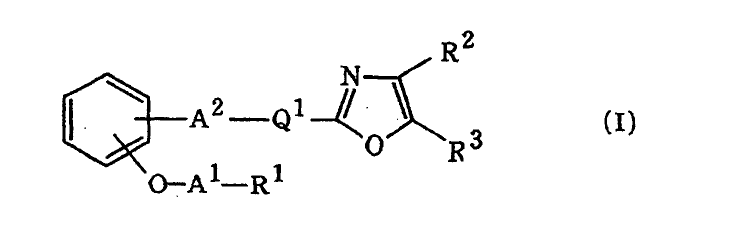 wherein R¹ represents carboxy or protected carboxy;
R² represents aryl which may have one or more suitable substituents;
R³ represents aryl which may have one or more suitable substituents;
A¹ represents lower alkylene;
A² represents singlebond, orloweralkylenewhichmayhavehydroxy or protected hydroxy;
-Q¹- represents any of the following: 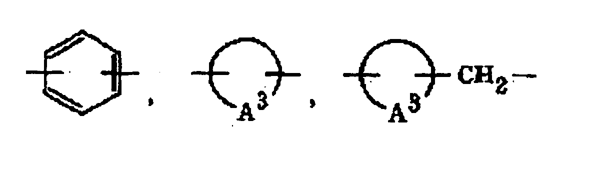 or 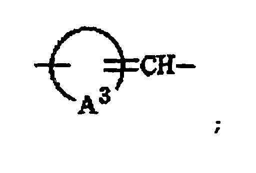 (in which 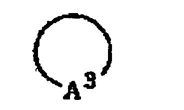 represents cyclo-lower alkane or cycle-lower alkene, which may have one or more suitable substituents.

Compounds of formula (II): 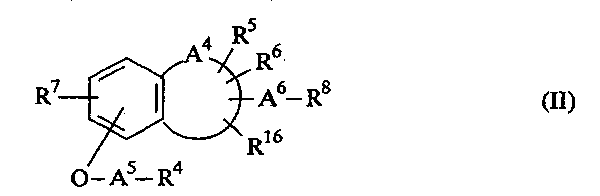 wherein R⁴ represents carboxy or protected carboxy;
R⁵ and R⁶ each represent hydrogen, hydroxy or protected hydroxy, or they may together form oxy or lower alkylene;
R¹⁶ represents hydrogen, hydroxy, protected hydroxy, lower alkyl or halogen;
R⁷ represents hydrogen or halogen;
A⁵ represents lower alkylene;
A⁶ represents single bond or lower alkylene;
-R⁸ represents 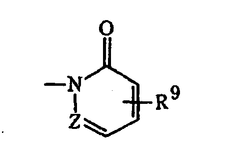 (in which R⁹ represents mono (or di or tri)-aryl-lower alkyl; Z represents N or CH)
or 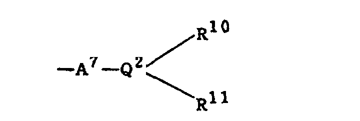 (in which -A⁷- represents 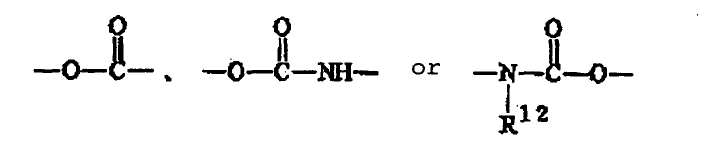 (R¹² represents hydrogen or lower alkyl) ; Q² represents N or CH; R¹⁰ and R¹¹ each represent aryl may have one or more suitable substituents; 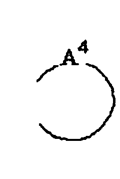 represents

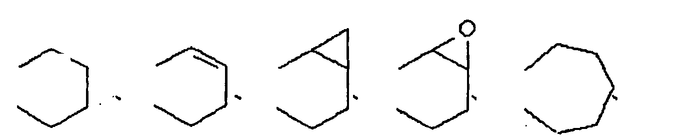

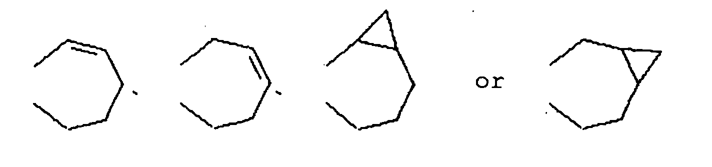

Compounds of formula (III): 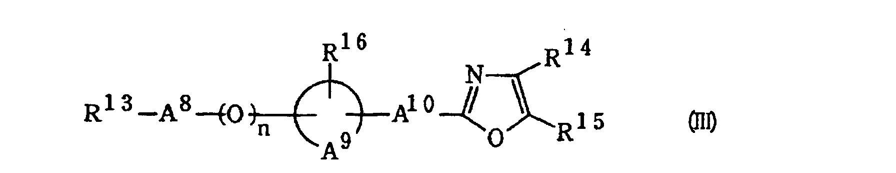 wherein R¹³ represents carboxy or protected carboxy;
R¹⁴ represents aryl which may have one or more suitable substituents;
R¹⁵ represents aryl which may have one or more suitable substituents;
R¹⁶ represents hydrogen, lower alkyl, hydroxy or aryl;
A⁸ represents lower alkylene; 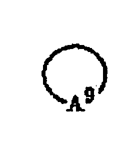 represents 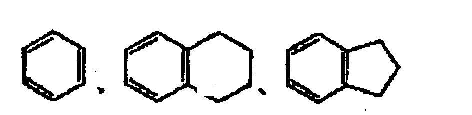 or 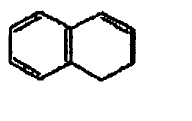 - A¹⁰- represents 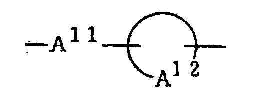 (in which -A¹¹- represents single bond, -CH₂-, or -CO-; 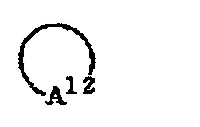 represents cyclo(C5-C8)alkene, cyclo(C7-C8)alkane, bicycloheptane, bicycloheptene, tetrahydrofuran, tetrahydrothiophene, azetidine, pyrrolidine or piperidine, each of which may have one or more suitable substituents; or -X-A¹³- (in which -X- represents -O-, -S-, or -N (R¹⁷) - (where R¹⁷ represents hydrogen, lower alkyl or acyl); A¹³ represents lower alkylene which may have one or more suitable substituents); n indicates 0 or 1.

Pharmaceutically-acceptable salts of the compounds of formulae (I) to (III) are any ordinary non-toxic salts, including, for example, metal salts such as alkali metal salts (e.g., sodium salts, potassium salts), alkaline earth metal salts (e.g., calcium salts, magnesium salts); ammonium salts; organic base salts (e.g., trimethylamine salts, triethylamine salts, pyridine salts, picoline salts, dicyclohexylamine salts, N,N'-dibenzylethylenediamine salts), organic acid salts (e.g., acetates, maleates, tartrates, methanesulfonates, benzenesulfonates, formates, toluenesulfonates, trifluoroacetates), inorganic acid salts (e.g._{;} hydrochlorides, hydrobromides, sulfates, phosphates), salts with amino acids (e.g., arginine, aspartic acid, glutamic acid).

The compounds of formulae (I) to (III) and their pharmaceutically-acceptable salts may have one or more asymmetric centers, and therefore they may include their enantiomers or diastereomers. The invention encompasses all of their mixtures and individual isomers.

The compounds of formulae (I) to (III) and their pharmaceutically-acceptable salts may be in the form of their solvates, which are within the scope of the invention. Preferred solvates of the compounds are hydrates and ethanolates thereof.

Preferred examples and concrete examples of the definitions which the invention encompasses in its scope and which are given hereinabove and hereinunder in this description are described in detail.

The term "lower" is meant to indicate from 1 to 6 carbon atoms, unless otherwise specifically indicated.

Preferred "aryl" and preferred "aryl moiety" in the expression of "mono (or di or tri)-aryl-lower alkyl" include phenyl, naphthyl.

Preferred "lower alkylene" is linear or branched, having from 1 to 6 carbon atoms. It includes, for example, methylene, ethylene, trimethylene, tetramethylene, pentamethylene, hexamethylene. More preferred are those having from 1 to 3 carbon atoms.

Preferred lower alkyl" andpreferred "lower alkylmoiety" in the expression of "mono (or di or tri)-aryl-lower alkyl" are linear or branched, having from 1 to 6 carbon atoms. It includes, for example, methyl, ethyl, propyl, isopropyl, butyl, isobutyl, secondary butyl, tertiarybutyl, pentyl, tertiarypentyl, hexyl. More preferred are those having from 1 to 4 carbon atoms.

Preferred "protected carboxy" is esterified carboxy.

Preferred examples of the ester moiety in the esterified carboxy includes;
(1) lower alkyl (e. g. , methyl, ethyl, propyl, isopropyl, butyl, isobutyl, tertiary butyl, pentyl, hexyl), optionally having at least one suitable substituent, for lower alkanoyloxy-lower alkyl [e.g., acetoxymethyl, propionyloxymethyl, butyryloxymethyl, valeryloxymethyl, pivaloyloxymethyl, hexanoyloxymethyl, 1 (or 2)-acetoxyethyl, 1 (or 2 or 3)-acetoxypropyl, 1 (or 2 or 3 or 4)-acetoxybutyl, 1 (or 2)-propionyloxyethyl, 1 (or 2 or 3)-propionyloxypropyl, 1 (or 2)-butyryloxyethyl, 1 (or 2)-isobutyryloxyethyl, 1 (or 2)-pivaloyloxyethyl, 1 (or 2)-hexanoyloxyethyl, isobutyryloxymethyl, 2-ethylbutyryloxymethyl, 3,3-dimethylbutyryloxymethyl,. 1 (or 2)-pentanoyloxyethyl], lower alkylsulfonyloxy-lower alkyl (e.g., 2-mesylethyl), mono (or di or tri)-halo-lower alkyl (e.g., 2-iodoethyl, 2,2,2-trichloroethyl), lower alkoxycarbonyloxy-lower alkyl (e.g., methoxycarbonyloxymethyl, ethoxycarbonyloxymethyl, 2-methoxycarbonyloxyethyl, 1-ethoxycarbonyloxyethyl, 1-isopropoxycarbonyloxyethyl), phthalidylidene-lower alkyl, (5-lower alkyl-2-oxo-1,3-dioxol-4-yl)-lower alkyl (e.g., 5-methyl-2-oxo-1,3-dioxol-4-yl)methyl, (5-ethyl-2-oxo-1,3-dioxol-4-yl)methyl, (5-propyl-2-oxo-1,3=dioxol-4-yl)ethyl];
(2) lower alkenyl (e.g., vinyl, allyl);
(3) lower alkynyl (e.g., ethynyl, propynyl);
(4) ar-lower alkyl optionally having at least one suitable substituent such as mono (or di or tri)-phenyl-lower alkyl optionally having at least one suitable substituent (e.g.,benzyl, 4-methoxybenzyl,4-nitrobenzyl,phenethyl,trityl,benzhydryl, bis(methoxyphenyl)methyl, 3,4-dimethoxybenzyl, 4-hydroxy-3,5-di-tert-butylbenzyl);
(5) aryl optionally having at least one suitable substituent (e.g., phenyl, 4-chlorophenyl, tolyl, tert-butylphenyl, xylyl, mesityl, cumenyl); and
(6) phthalidyl.

Preferred "substituents" in the expression of "aryl which may have one or more suitable substituents" include halogen, amino, hydroxy, lower alkoxy, and lower alkyl such as those mentioned hereinabove.

Preferred ''cyclo-lower alkane" includes cyclopropane, cyclobutane, cyclopentane, cyclohexane.

Preferred "cyclo-lower alkene" includes cyclopropene, cyclobutene, cyclopentene, cyclohexene.

Preferred "substituents" in the expression of "cyclo-lower alkane or cycle-lower alkene, which may have one or more suitable substituents" include epoxy, hydroxyl and lower alkoxy.

Preferred "lower alkoxy" includes methoxy, ethoxy, propoxy, isopropoxy, butoxy, isobutoxy, tertiary butoxy, pentyloxy, tertiary pentyloxy, hexyloxy.

Preferred "protected hydroxy" includes acyloxy. Preferred "acyl" and preferred "acyl moiety" in the expression of "acyloxy" include aliphatic acyl group and acly group having aromatic ring or heterocyclic ring.

Preferred examples of the acyl are; lower alkanoyl (e.g., formyl, acetyl, propionyl, butyryl, isobutyryl, valeryl, isovaleryl, oxalyl, succinyl, pivaloyl);
lower alkoxycarbonyl (e.g., methoxycarbonyl, ethoxycarbonyl, propoxycarbonyl, isopropoxycarbonyl, butoxycarbonyl, tertiary butoxycarbonyl, pentyloxycarbonyl, hexyloxycarbonyl);
lower alkylsulfonyl (e.g., mesyl, ethanesulfonyl, propanesulfonyl, isopropanesulfonyl, butanesulfonyl);
arenesulfonyl (e.g., benzenesulfonyl, tosyl);
aroyl (e.g., benzoyl, toluoyl, xyloyl, naphthoyl, phthaloyl, indanecarbonyl);
ar-lower alkanoyl (e.g., phenylacetyl, phenylpropionyl);
ar-lower alkoxycarbonyl (e.g., benzyloxycarbonyl, phenethyloxycarbonyl).

Preferred "halogen" includes chlorine, bromine, iodine, fluorine.

Preferred "cyclo(C5-C8)alkene" includes cyclopentene, cyclohexene, cyclopentene, cyclooctene.

Preferred "cyclo(C7-C8)alkane" includes cycloheptane, cyclooctane.

Preferred "bicycloheptane" includes bicyclo[2.2.1]heptane.

Preferred "bicycloheptene" includes bicyclo[2.2.1]heptene (e.g., bicyclo[2.2.1]hept-2-ene).

Preferred "substituents" in the expression of "cyclo(C5-C8)alkene, cyclo(C7-C8)alkane, bicycloheptane, bicycloheptene, tetrahydrofuran, tetrahydrothiophene, azetidine, pyrrolidine or piperidine, each of which may have one or more suitable substituents" include imino, hydroxy, oxo, acyl such as those mentioned hereinabove, and imino-protective group.

Preferred "imino-protective group" includes mono (or di or tri)-aryl-lower alkyl.

Preferred "substituents" in the expression of "lower alkylene which may have one or more suitable substituents" include lower alkyl such as those mentioned hereinabove, hydroxy-lower alkyl (e.g., hydroxymethyl., hydroxyethyl, hydroxypropyl, hydroxybutyl, hydroxypentyl, hydroxyhexyl).

Preferred examples of the compounds of formula (I) are mentioned below.
R¹ represents carboxy or protected carboxy (more preferably esterified carboxy, even more preferably lower alkoxycarbonyl) ;
R² represents aryl which may have from 1 to 3 (more preferably one) suitable substituents (more preferably phenyl or lower alkylphenyl);
R³ represents aryl which may have from 1 to 3 (more preferably one) suitable substituents (more preferably phenyl or lower alkylphenyl);
A¹ represents lower alkylene (more preferably C1-C3 alkylene, even more preferably methylene);
A² represents single bond, or lower alkylene (more preferably C1-C3 alkylene, even more preferably methylene);
-Q¹- represents 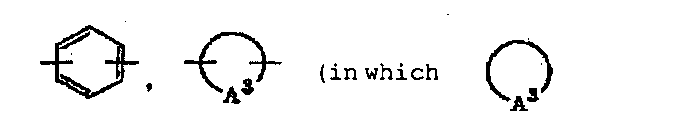 represents cyclo-lower alkane or cyclo-lower alkene, which may have one substituent selected from epoxy, hydroxy and lower alkoxy;
or 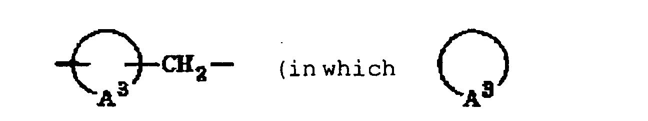 represents cyclo-lower alkane or cyclo-lower alkene which may have one substituent selected from epoxy and hydroxy;
or 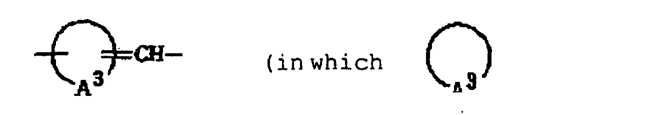 represents cyclo-lower alkane.

Preferred examples of the compounds of formula (II) are mentioned below.
R⁴ represents carboxy or protected carboxy (more preferably esterified carboxy,even more preferablyloweralkoxycarbonyl);
R⁵ represents hydrogen, hydroxy or protected hydroxy (more preferably acyloxy);
R⁶ represents hydrogen, hydroxy, protected hydroxy (more preferably acyloxy), lower alkyl or halogen;
A⁵ represents lower alkylene (more preferably C1-C3 alkylene, even more preferably methylene);
A⁶ represents single bond, or lower alkylene(more preferably C1-C3 alkylene, even more preferably methylene or ethylene);
-R⁸ represents 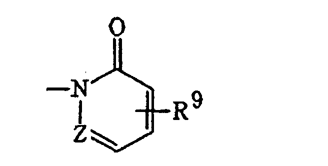 in which R⁹ represents diaryl-lower alkyl (more preferably diphenyl-lower alkyl, even more preferably diphenylmethyl); Z represents N or CH;
or 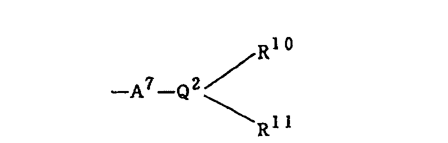 in which -A⁷- represents 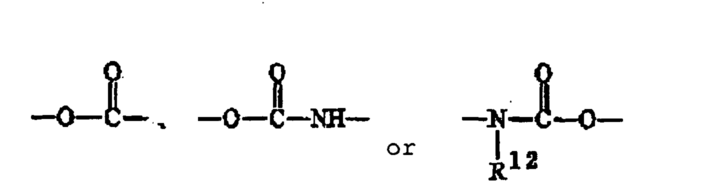 R¹² represents hydrogen or lower alkyl;
Q² represents N or CH;
R¹⁰ represents aryl (more preferably phenyl);
R¹¹ represents aryl (more preferably phenyl); 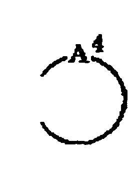 represents

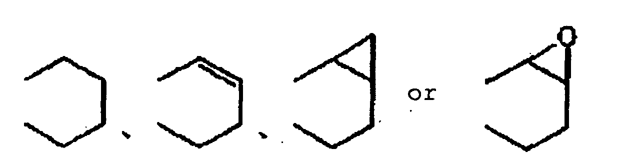

Preferred examples of the compounds of formula (III) are mentioned below.
R¹³ represents carboxy or protected carboxy (more preferably esterified carboxy, even more preferably lower alkoxycarbonyl) ;
R¹⁴ represents aryl optionally having lower alkyl (more preferably phenyl or lower alkylphenyl, even more preferably phenyl or C1-C4 alkylphenyl);
R¹⁵ represents aryl optionally having lower alkyl (more preferably phenyl or lower alkylphenyl, even more preferably phenyl or C1-C4 alkylphenyl);
R¹⁶ represents hydrogen, lower alkyl (more preferably C1-C4 alkyl, even more preferably methyl), hydroxy, or aryl (more preferably phenyl);
A⁸ represents lower alkylene (more preferably C1-C4 alkylene, even more preferably methylene or ethylene); 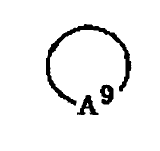 represents 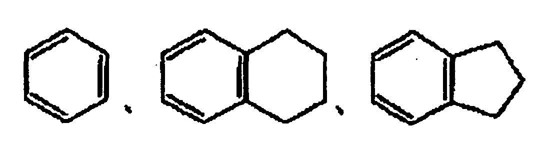 or 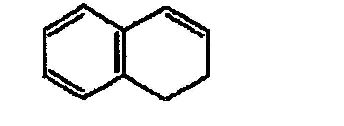 -A¹⁰- represents
in which -A¹¹- represents single bond, -CH₂-, or -CO-; 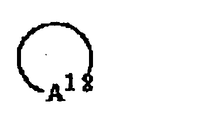 represents cyclo(C5-C8)alkene, cyclo(C7-C8)alkane, bicycloheptane (more preferably bicyclo[2.2.1]heptane), bicycloheptene (more preferably bicyclo[2.2.1]heptene, even more preferably bicyclo[2.2.1]hept-2-ene), tetrahydrofuran, tetrahydrothiophene, azetidine, pyrrolidine or piperidine, each optionally having from 1 to 3 (more preferably one or two) suitable substituents selected from a group consisting of imino, oxo, acyl (more preferably lower alkanoyl, even more preferably C1-C4 alkanoyl) and imino-protective group (morepreferablymono (or di or tri)phenyl-lower alkyl, even more preferably phenyl-lower alkyl);
or -X-A¹³- (in which -X- represents -O-, -S-, or -N (R¹⁷) - where R¹⁷ represents hydrogen, lower alkyl (more preferably C1-C4 alkyl), or acyl (more preferably lower alkanoyl, even more preferably C1-C4 alkanoyl); A¹³ represents lower alkylene (more preferably C1-C4 alkylene, even more preferably methylene or ethylene) optionally having from 1 to 3 (more preferably one) suitable substituents selected from a group consisting of lower alkyl (morepreferablyC1-C4 alkyl) and hydroxy-lower alkyl (more preferably hydroxy-C1-C4 alkyl));
n indicates 0 or 1.

More preferred compounds of formula (III) are those of the following formula (III-A): 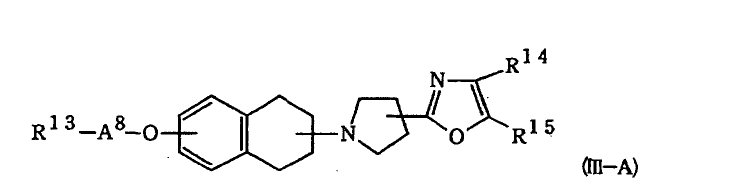 wherein R¹³ represents carboxy, or protected carboxy (more preferably esterified carboxy, even more preferably lower alkoxycarbonyl);
R¹⁴ represents phenyl, or lower alkylphenyl (more preferably C1-C4 alkylphenyl);
R¹⁵ represents phenyl, or lower alkylphenyl (more preferably C1-C4 alkylphenyl);
A⁸ represents lower alkylene (more preferably C1-C4 alkylene, even more preferably methylene).

More preferred prostaglandin-I₂ agonists for use in the invention are
[3-[[(1S)-2-(4,5-diphenyloxazol-2-yl)-2-cyclohexen-1-yl]methyl]phenoxy]acetic acid,
[3-[[(1S)-2-(4,5-diphenyloxazol-2-yl)-2-cyclopenten-1-yl]methyl]phenoxy]acetic acid,
[[(2R)-5-(carboxymethoxy)-2-hydroxy-1,2,3,4-tetrahydronaphth-2-yl]methyl] N,N-diphenylcarbamate,
(1R)-1-[(2R)-2-(4,5-diphenyloxazol-2-yl)pyrrolidin-1-yl)-5-carboxymethoxy-1,2,3,4-tetrahydronaphthalene,
[3-[[(2R)-2-(4,5-diphenyloxazol-2-yl)pyrrolidin-1-yl]methyl]phenoxy]acetic acid and their salts.

The compounds of formulae (I), (II) and (III) and the specific compounds mentioned above are known, and can be prepared according to the methods described in the following publications or in the same manner as therein (these patent publications are referred to herein as a part of this description).
International Publication No. WO 95/17393
International Publication No. WO 95/24393
International Publication No. WO 97/03973
International Publication No. WO 99/21843
International Publication No. WO 99/24397
International Publication No. WO 99/32435
International Publication No. WO 01/16132

Other preferred examples of the prostaglandin-I₂ agonist for use in the invention are the following:
(1) Condensed benzeneoxy-acetic acid derivatives described in EuropeanpatentLaid-OpenNos. EP578847A, EP548949A, EP542203A1, EP581187A and EP558062A (these patent publications are referred to herein as a part of this description), preferably a compound of the following formula (IV) and its salts: 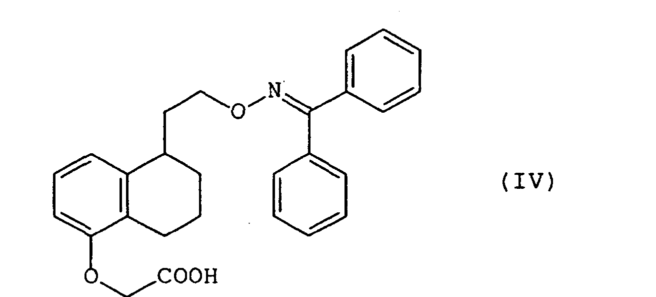
(2) Phenoxyacetic acid derivatives described in USP 5, 348, 969 (this patent publication is referred to herein as a part of this description), preferably a compound of the following formula (V) and its salts: 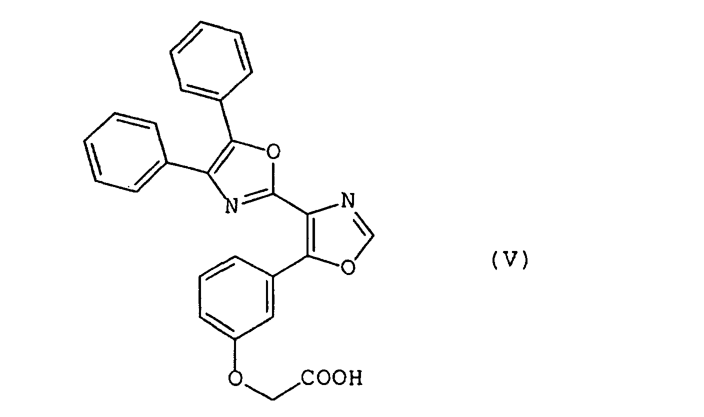
(3) Tricyclic compounds described in International Laid-Open No. WO98/13356 (this patent publication is referred to herein as a part of this description), preferably a compound of the following formula (VI) and its salts:

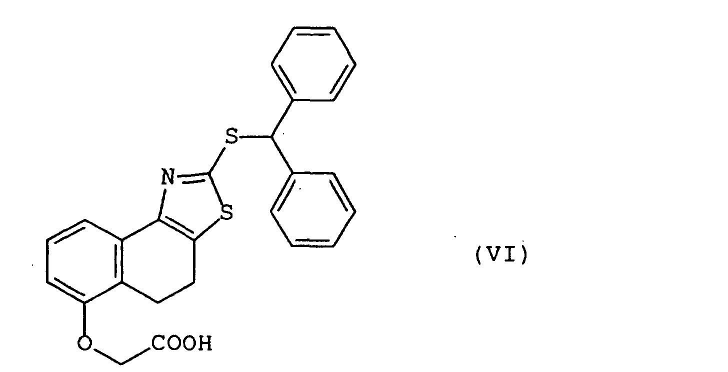

The pharmaceutical composition of the invention is used as medicines, for example, as solid, semisolid or liquid medicines suitable for rectal, transpulmonary (nasal or oral inhalation), nasal, ophthalmic, external (local), oral or parenteral (including subcutaneous, intravenous and intramuscular) administration or inhalation (for example, tablets, pellets, troches, capsules, suppositories, creams, ointments, aerosols, powders, liquids, emulsions, suspensions).

The pharmaceutical composition of the invention may contain various organic or inorganic carrier substances generallyemployedinpharmaceutics, for example,vehicles (e.g., sucrose, starch, mannitol, sorbitol, lactose, glucose, cellulose, talc, calcium phosphate, calcium carbonate), binder (e.g., cellulose, methyl cellulose, hydroxypropyl cellulose, polypropylpyrrolidone, gelatin, gum arabic, polyethylene glycol, sucrose, starch), disintegrator (e.g., starch, carboxymethyl cellulose, calcium carboxymethyl cellulose, hydroxypropyl starch, sodium starch glycolate, sodium hydrogencarbonate, calcium phosphate, calcium citrate), lubricant (e.g., magnesium stearate, talc, sodium laurylsulfate) , flavoring (e. g., citric acid, menthol, glycine, bitter orange), preservative (e.g., sodium benzoate, sodium hydrogensulfite, methylparaben, propylparaben), stabilizer (e.g., citric acid, sodium citrate, acetic acid), suspending agent (e.g., methyl cellulose, polyvinylpyrrolidone, aluminium stearate), dispersant, aqueous diluent (e.g., water), base wax (e.g., cacao butter, polyethylene glycol, white petrolatum).

The dose of the active ingredient may be generally from 0.01 mg/kg to 50 mg/kg, and it may be administered once to four times a day. However, the dose may vary depending on the age, the body weight and the condition of the case to which it is administered and on the administration route.

### EXAMPLES

The following is to demonstrate the fibrogenesis inhibiting activity of prostaglandin-I₂ agonist. The following test compound, a typical example of the agonist is used herein. Its prostaglandin-I₂ agonist effect in dimethylnitrosamine-induced rat hepatitis models and in bleomycin-induced mouse fibrotic lung models was investigated.

### [Test Compound]

(1) [[(2R)-5-(carboxymethoxy)-2-hydroxy-1,2,3,4-tetrahydronaphth-2-yl]methyl] N,N-diphenylcarbamate (hereinafter referred to as "test compound").

### Test Example 1 (Effect of test compound for inhibiting hepatic injury and fibrogenesis in dimethylnitrosamine-induced rat hepatitis models):

Dimethylnitrosamine was frequently administered to rats for 3 weeks to thereby make the rats have irreversible hepatic fibrogenesis. The rats are the test models in this experiment. As the index of hepatic injury and fibrogenesis, the hepatic hydroxyproline was measured in every rat and the liver tissue of each rat was pathologically analyzed. Based on the data, the tissue fibrogenesis-inhibiting effect of the prostaglandin-I₂ agonist was evaluated.

### [Test Method]

10 mg/kg of dimethylnitrosamine was intra-abdominally administered to rats everyday in continuous 3 days a week, over a period of three weeks. 5 days after the final administration of dimethylnitrosamine, the liver was taken out of each rat and its weight was measured. Then, distal potion of the left lobe of the liver was cut out. A part of it was used for histologic analysis^{*1)} for hepatic fibrogenesis; and another part thereof was used for determination of hepatic hydroxyproline content^{*2)}.

The test compound was orally administered to the rats from the initial dimethylnitrosamine administration to one daybefore the blood collection from the rats. On the day for dimethylnitrosamine administration, the test compound was administered 12 hours after the dimethylnitrosamine administration; and on the other day, it was administered once a day. The effect of the test compound for curing hepatic injury and fibrogenesis was investigated. The test compound was administered to three groups - 1 mg/kg/day administration group (n = 15), 3.2 mg/kg/day administration group (n = 15), and 10 mg/kg/day administration group (n = 15). To the comparative control group, 0 mg/kg administration group (n = 15), a solvent was administered in place of the test compound. To confirm the presentation of hepatic fibrogenesis through dimethylnitrosamine administration, a solvent in place of dimethylnitrosamine was administered to a no-treatment group (n = 5).

### 1) Histological analysis for hepatic fibrogenesis:

A distal potion of the left lobe of the liver was fixed in 10 % neutral buffer formalin liquid, then stained with azan to prepare an anatomicopathological specimen, on which the degree of fibrogenesis was classified into 5 grades according to the fibrogenesis criteria mentioned below.
Grade 0: No fibrogenesis was found.
Grade 1: Light fibrogenesis was found only locally in and around the portal vein and the central vein.
Grade 2: The fibrogenesis in and around the portal vein and the central vein extended to the lobe.
Grade 3: Noticeable fibrogenesis was found to fragment the lobe.
Grade 4: High-level fibrogenesis was found along with pseudo lobe formation.

### 2) Determination of hepatic hydroxyproline:

A part of the left lobe of the liver that had been frozen at -80°C was thawed, and 1 ml/500 mg-liver weight of phosphate balanced salt buffer (PBS (-), Dulbecco PBS (-)) was added to it. Then, this was homogenized in Polytron (power control 7, type; PT 10 20 350D, by KINEMATICA) for 1 minute. 4 ml of the thus-homogenized liquid was transferred into a screw cap-equipped centrifuge (by PYREX), and 0.4 ml of 9M HCl was added to it and incubated at 120°C for 8 hours (in DRYING STERILIZER SG-62, by YAMATO). Next, this was further incubated at 4°C overnight, then neutralized with 6 M KOH and 9M HCl, and then filtered (pore size 0.45 µm, MILLIPORE) . 1ml of chloramine-T^{(**)} was added to 100 µl of the filtrate or a standard liquid^{(*)}, and left at room temperature for 20 minutes. 1 ml of Ehrich solution^{(***)} was added to it, and incubated at 65°C for 40 minutes. The reaction was stopped in ice-water, and then the absorbance (OD560) of the reaction liquid was measured with an spectrophotometer UV2100PC (UV-VIS SCANNING SPECTROPHOTOMETER, by SHIMADZU).

### Standard liquid:

Hydroxy-L-proline (by NACALAI TESQUE) was dissolved in physiological saline (8 mg/ml) , and freeze-dried. Before use, it was thawed and diluted with physiological saline into 5, 10, 20, 40, 80 µg/ml standard solution.

| Chloramine-T liquid: (50 ml preparation) | |
|---|---|
| Chloramine T (by TOKYO CHEMICAL) | 0.71g |
| N-propanol (by NACALAI TESQUE) | 5 ml |
| Distilled water | 5 ml |
| Citrate/acetate buffer | 40 ml |

| Ehrich solution: (50 ml preparation) | |
|---|---|
| P-dimethylaminobenzaldehyde (by NACALAI TESQUE) | 8.53 g |
| 1-Propanol (NACALAI TESQUE) | 35.2 ml |
| 60 % Perchloric acid (NACALAI TESQUE) | 14.8 ml |

| Citrate/acetate buffer: (500 ml preparation) | |
|---|---|
| Citrate acid anhydrous (by HAYASHI PURE CHEMICAL) | 25 g |
| Sodium acetate trihydrate (by NACALAI TESQUE) | 60 g |
| NaOH (by KANTO CHEMICAL) | 17 g |
| Acetic acid (by KANTO CHEMICAL) to make pH of about 6.0 | |
| Distilled water | 500 ml |

### [Test Result]

The result of histological investigation of hepatic fibrogenesis is given in Table 1.

**[Table 1]**

| Result of Histological Investigation of Hepatic Fibrogenesis | | |
|---|---|---|
| Administration Group (mg/kg) | Number of Animals | Hepatic Fibrogenesis Score |
| No-treatment group | 5 | 0.0 ± 0.0^{**} |
| Test compound (0 mg/kg) | 15 | 2.1 ± 0.2 |
| Test compound (1 mg/kg) | 15 | 1.7 ± 0.2 |
| Test compound (3.2 mg/kg) | 15 | 1.3 ± 0.2^{*} |
| Test compound (10 mg/kg) | 15 | 1.3 ± 0.2^{*} |

| | | |
|---|---|---|
| *p<0.05, **p<0.01 vs. 0 mg/kg | | |

As is obvious from the result in Table 1, hepatic fibrogenesis was found in the dimethylnitrosamine-administered control group, different from that in no-treatment group with no dimethylnitrosamine administration. On the other hand, hepatic fibrogenesis was inhibited in the test compound-administered groups.

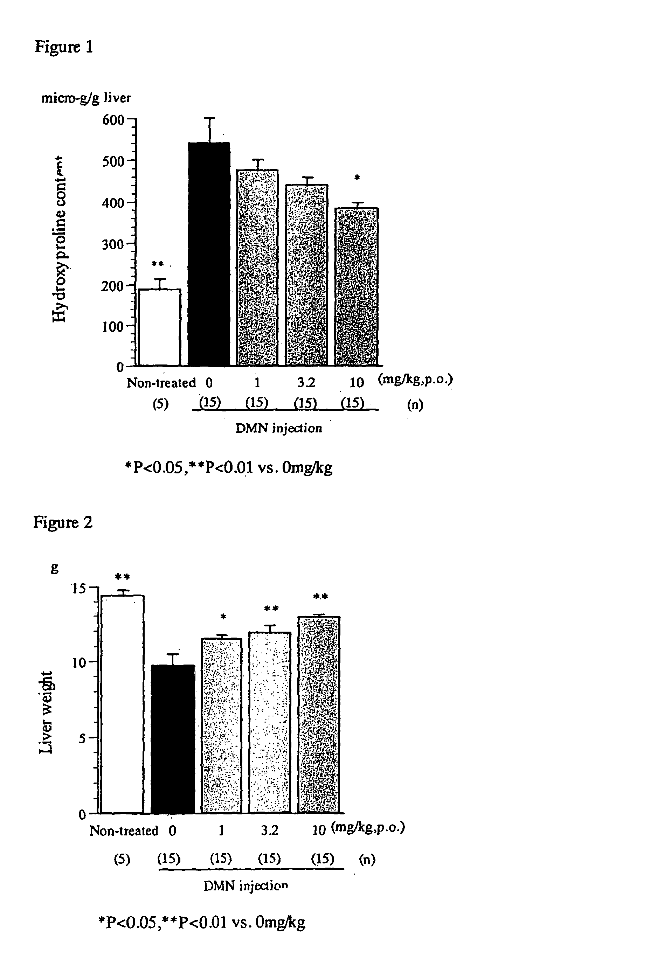

As a quantitative index of the degree of seriousness of hepatic fibrogenesis, the hepatic hydroxyproline content (hepatic collagen content) was measured. The result is shown in Fig. 2. In the dimethylnitrosamine-administered control group, the hepatic hydroxyproline content increased as compared with that in the no-treatment group with no dimethylnitrosamine administration. On the other hand, the hepatic hydroxyproline content decreased in the test compound-administered groups. Accordingly, the test compound inhibited the irreversible hepatic fibrogenesis in the models.

The liver weight data are shown in Fig. 3. In the dimethylnitrosamine-administered control group, the liver weight decreased as compared with that in the no-treatment group with no dimethylnitrosamine administration. On the other hand, the liver weight reduction was suppressed in the test compound-administered groups. Accordingly, the test compound promoted the liver generation in the models.

### Test Example 2 (Effect of test compound in bleomycin-induced lung fibrosis model in mice):

Bleomycin was frequently administered to mice for 10 days to thereby make the mice fibrotic lung. The mice are the test models in this experiment. As the index of lung fibrogenesis, the lung hydroxyproline was measured in every mouse, from which the effect of the prostaglandin-I₂ agonist for inhibiting lung fibrogenesis was investigated.

### [Test Method]

1 . 10 mg/kg of bleomycin that had been dissolved in saline to have a concentration of 1 mg/ml was intraperitoneally administered to 8-weeks-old male C57BL/6N mice, once a day, everyday for continuous 10 days. Saline alone was intraperitoneally administered to the negative control group.
2. To the animals of the positive control group and the test compound-administered group, 10 ml/kg of pure water or an aqueous solution of the test compound (32 mg/kg) was orally administered everyday during the test period from the start of the bleomycin administration.
3. 4 weeks after the start of the bleomycin administration, the animals were killed in a mode of euthanasia, the lung was taken out, its wet weight was measured, and the lung was then freesed at -30°C.
4. The freeze-dried lung was thawed, PBS (2 ml) was added. to it, and this was homogenized by a homogenizer on ice. To 0.4 ml of the resulting homogenate, the same amount of concentrated hydrochloric acid (36% hydrochloric acid) was added, and hydrolyzed under heat at 120°C for 8 hours.
5. The hydrolyzed sample was neutralized with 6 mols/liter of potassium hydroxide, and the hydroxyproline content of the sample was measured, from which the lung hydroxyproline content was derived. This indicates the lung collagen content and the degree of lung fibrogenesis.

### [Test Result]

As a quantitative index of the degree of seriousness of lung fibrogenesis, the lung hydroxyproline content (lung collagen content) was measured. The result is given in Table 2.

**[Table 2]**

| Test Group | n | Hydroxyproline Content (ug/lung) |
|---|---|---|
| Negative Control | 10 | 200.03 ± 6.9707 |
| Positive Control | 14 | 398.19 ± 15.483 |
| Test Compound 32 mg/kg | 15 | 308.37 ± 19.403^{**} |

| | | |
|---|---|---|
| **: p<0.01 vs positive control group (Mann-Whitney U Test) | | |

Oral administration of 32 mg/kg of the test compound significantly inhibited the increase in the lung hydroxyproline content caused by bleomycin administration.

As in the above results, the test compound inhibited fibrogenesis in liver and lung in the hepatic or lung tissue fibrogenesis models. As a result, it is believed that the compound improves the balance between the hepatic growth and the fibrogenesis in live and is therefore effective for restoring to normal liver. In lung, it is also believed that the compound inhibits abnormal tissue fibrogenesis after lung tissue damage.

### INDUSTRIAL APPLICABILITY

As described in detail hereinabove, the pharmaceutical composition that contains, as the active ingredient thereof, a prostaglandin-I₂ agonist inhibits hepatic and lung tissue fibrogenesis. Accordingly, it will be extremely effective in clinical use as a remedy and/or a preventive for hepathopathy such as acute or chronic hepatitis, cirrhosis and fatty liver that may be caused by viral infection, alcohol, chemicals or autoimmunity diseases, and as a remedy and/or a preventive for interstitial pneumonia and fibroid lung (for example, that follows pulmonary emphysema) . Further, owing to its effect of inhibiting fibrogenesis, the pharmaceutical composition is extremely effective for treatment and prevention of various organ disorders caused by the progress in tissue fibrogenesis in kidney, pancreas and skin (for example, pancreatitis with fibrogenesis, nephritis with fibrogenesis).

### BRIEF DESCRIPTION OF THE DRAWINGS

Fig. 1 is a graph showing the effect for the hepatic hydroxyproline content described in [Pharmaceutical Test I].
Fig. 2 is a graph showing the effect for the liver weight described in [Pharmaceutical Test I].

## Claims

1. A pharmaceutical composition for treatment or prevention of disorders with tissue fibrogenesis in humans and animals, which contains, as the active ingredient thereof, a prostaglandin-I₂ agonist.

2. A method for inhibiting tissue fibrogenesis, which comprises administering a therapeutically effective amount of a prostaglandin-I₂ agonist to a patient with tissue fibrogenesis.

3. A method of using a prostaglandin-I₂ agonist in producing a pharmaceutical composition for treatment. or prevention of disorders with tissue fibrogenesis.

4. A pharmaceutical composition for treatment or prevention of hepatic disorders with fibrogenesis in humans and animals, which contains, as the active ingredient thereof, a prostaglandin-I₂ agonist.

5. A pharmaceutical composition for prevention of cirrhosis or hepatic insufficiency in humans and animals, which contains, as the active ingredient thereof, a prostaglandin-I₂ agonist.

6. A pharmaceutical composition for treatment or prevention of lung disorders with fibrogenesis in humans and animals, which contains, as the active ingredient thereof, a prostaglandin-I₂ agonist.

7. A pharmaceutical composition for treatment or prevention of fibroid lung in humans and animals, which contains, as the active ingredient thereof, a prostaglandin-I₂ agonist.

8. The pharmaceutical composition as claimed in any of claims 1, and 4 to 7, wherein the prostaglandin-I₂ agonist is a compound of the following formula (I) or its pharmaceutically-acceptable salt: 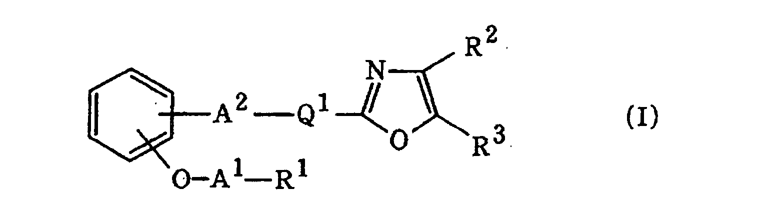 wherein R¹ represents carboxy or protected carboxy;
R² represents aryl which may have one or more suitable substituents;
R³ represents aryl which may have one or more suitable substituents;
A¹ represents lower alkylene;
A² represents single bond, or lower alkylene which may have hydroxy or protected hydroxy; and
-Q¹- represents any of the following 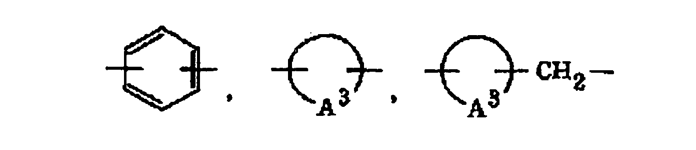 or 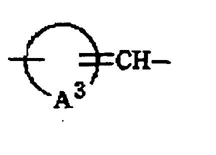 (in which 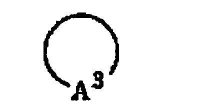 represents cyclo-lower alkane or cyclo-lower alkene, which may have one or more suitable substituents).

9. The pharmaceutical composition as claimed in any of claims. 1, and 4 to 7, wherein the prostaglandin-I₂ agonist is a compound of the following formula (II) or its pharmaceutically-acceptable salt: 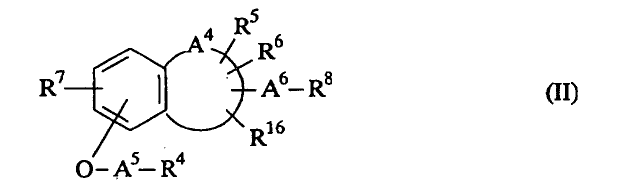 wherein R⁴ represents carboxy or protected carboxy;
R⁵ and R⁶ each represent hydrogen, hydroxy or protected hydroxy, or they may together-form oxy or lower alkylene;
R¹⁶ represents hydrogen, hydroxy, protected hydroxy, lower alkyl, or halogen;
R⁷ represents hydrogen or halogen;
A⁵ represents lower alkylene;
A⁶ represents single bond, or lower alkylene;
-R⁸ represents 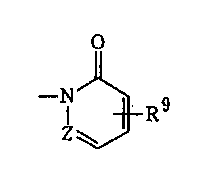 in which R⁹ represents mono (or di or tri)-aryl-lower alkyl;
Z represents N or CH;
or 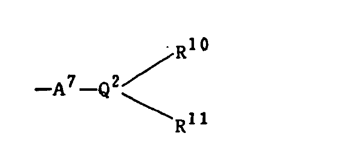 in which -A⁷- represents 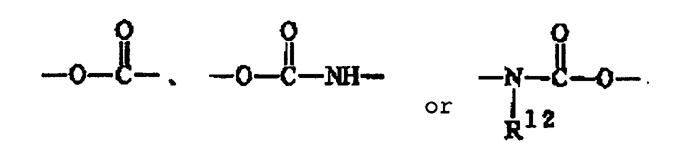 (R¹² represents hydrogen or lower alkyl);
Q² represents N or CH;
R¹⁰ and R¹¹ each represent aryl which may have one or more suitable substituents; and 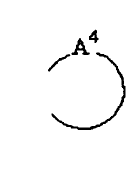 represents

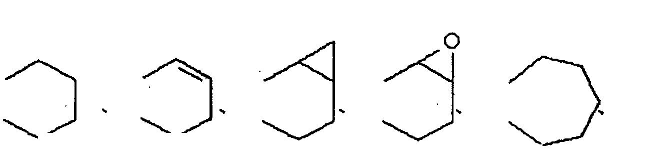

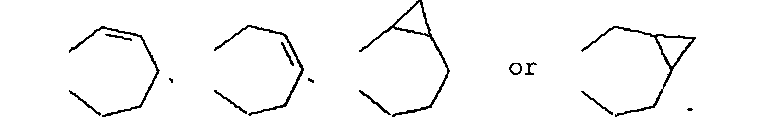

10. The pharmaceutical composition as claimed in any of claims 1, and 4 to 7, wherein the prostaglandin-I₂ agonist is a compound of the following formula (III) or its pharmaceutically-acceptable salt: 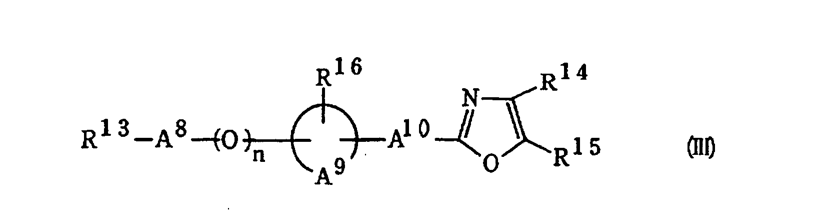 wherein R¹³ represents carboxy or protected carboxy;
R¹⁴ represents aryl which may have one or more suitable substituents;
R¹⁵ represents aryl which may have one or more suitable substituents;
R¹⁶ represents hydrogen, lower alkyl, hydroxy, or aryl;
A⁸ represents lower alkylene; 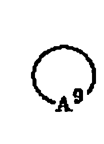 represents 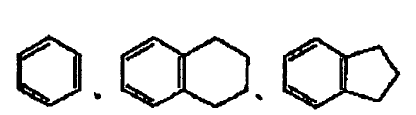 or 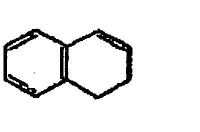 -A¹⁰- represents 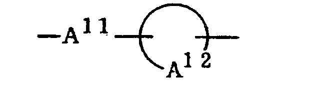 (in which -A¹¹- represents single bond, -CH₂-, or -CO-; 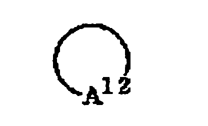 represents cyclo(C5-C8)alkene, cyclo(C7-C8)alkane, bicycloheptane, bicycloheptene, tetrahydrofuran, tetrahydrothiophene, azetidine, pyrrolidine or piperidine;
each of which may have one or more suitable substituents;
or -X-A¹³- (in which -X- represents -O-, -S-, or -N (R¹⁷) - where R¹⁷ represents hydrogen, lower alkyl, or acyl; A¹³ represents lower alkylene optionally having one or more suitable substituents); and
n indicates 0 or 1.

11. The pharmaceutical composition as claimed in any of claims 1, and 4 to 7, wherein the prostaglandin-I₂ agonist is:
(1) [3-[[(1S)-2-(4,5-diphenyloxazol-2-yl)-2-cyclohexen-1-yl]methyl]phenoxy]acetic acid,
(2) [3-[[(1S)-2-(4,5-diphenyloxazol-2-yl)-2-cyclopenten-1-yl]methyl]phenoxy]acetic acid,
(3) [[(2R)-5-(carboxymethoxy)-2-hydroxy-1,2,3,4-tetrahydronaphth-2-yl]methyl] N,N-diphenylcarbamate,
(4) (1R)-1-[(2R)-2-(4,5-diphenyloxazol-2-yl)pyrrolidin-1-yl]-5-carboxymethoxy-1.,2,3,4-tetrahydronaphthalene, or
(5) [3-[[(2R)-2-(4,5-diphenyloxazol-2-yl)pyrrolidin-1-yl]methyl]phenoxy]acetic acid,
or a pharmaceutically-acceptable salt thereof.
